# EUROPÄISCHE PATENTANMELDUNG

(11) **EP 0 911 394 A2**
(43) Veröffentlichungstag der Anmeldung: **28.04.1999**
(21) Anmeldenummer: 98116059.1
(22) Anmeldetag: 26.08.1998
(51) Int. Cl.: C12N 15/01

(54) **Verwendung von Aluminiumverbindungen**

(30) Priorität: 19.09.1997 DE 19741292
(71) Anmelder: GSF-Forschungszentrum für Umwelt und Gesundheit GmbH, 85764 Neuherberg (DE)
(72) Erfinder: Bengsch, Eberhard, Prof. Dr., 81549 München (DE); Kettrup, Antonius, Prof. Dr., 59821 Arnsberg (DE); Polster, Jürgen, Prof. Dr., 85356 Freising (DE)

(57) **Zusammenfassung**

Die Erfindung betrifft die Verwendung von Aluminium in Form von bioassimilierbaren Aluminiumverbindungen.

Aufgabe der Erfindung ist es, die natürlicherweise extrem seltene genetische Transposition in einen effizienten, steuerbaren, technisch verwertbaren Prozeß überzuführen und dabei Zelle, Organ und Organismus voll funktionsfähig zu halten.

Gelöst wird diese Aufgabe durch die Verwendung von Aluminium zur Regulierung der genetischen Transposition bei Pflanzen, Tieren, deren Zellkulturen und bei Mikroorganismen.

## Beschreibung

Die Erfindung betrifft eine neue Verwendung von Aluminium.

Normalerweise sind genetische Transpositionsereignisse in den Genomen von Pflanzen und Tieren (sowie deren Zellkulturen) und von Mikroorganismen extrem selten.

Die Transposition ist natürlicherweise ineffizient und technisch nicht anwendbar.

Transponierbare Elemente sind Nukleinsäureabschnitte, die innerhalb der perlschnurartig festliegenden Reihenfolge der Gene im Genom einer Spezies ihre Position wechseln können. Sie lösen sich aus Donorstellen und werden zu mehr oder weniger weit entfernten Zielorten in der DNA (kodierende und regulatorische Einheiten, Introns und andere nichtkodierende Bereiche) transponiert.

Dadurch wird die ursprüngliche Reihenfolge von Genen verändert. Es kommt zur Aktivierung und Inaktivierung von Genen. Gelegentlich treten als makroskopisch sichtbare Zeichen Chromosomenbrüche auf.

Als Gesamteffekt resultiert daraus eine Auflockerung der chromosomalen Ordnung. Die neue Merkmalsausbildung zeigt eine Verteilung, die mit der klassischen Genetik nicht in Einklang zu bringen ist.

Dies führte bereits 1947 (B. McClintock, Carnegie Inst. Washington - Year Book 46 (1947) 146-152) zur Entdeckung der Transposonen als damals noch hypothetische springende Gene: Unregelmäßige Pigmentierungsmuster an Maiskörnern (Bezirke mit oder ohne Anthocyansynthese) waren mit den Mendelschen Gesetzen unvereinbar. Hingegen waren sie beispielsweise mit mikroskopisch sichtbaren Chromosomenbrüchen an spezifischen Sollbruchstellen streng korreliert (Ac/Ds-System). Ohne Kenntnis der genetischen

Details wurden transpositionsbedingte Mutationen bereits viel früher von Humboldt und Darwin beschrieben. Jahrhundertealte Beschreibungen und bildhafte Darstellungen weisen auf die Beobachtung des Phänomens in noch früheren Zeiten hin.

Seit dem Start der wissenschaftlich-systematischen Beobachtungen von 1947 durch McClintock hat sich die Untersuchung der genetischen Transposition zu einem weitreichenden beschreibenden Wissenszweig entwickelt.

Den vielfältigen Anwendungsmöglichkeiten dieses Wissenspotentials stellte sich bisher ein grundsätzliches Hindernis blockierend entgegen: die extreme Seltenheit der transpositionalen Ereignisse in natürlichen Prozessen und die daraus resultierende quasi-totale Ineffizienz der Transposition unter natürlichen Lebensbedingungen der Zelle, wenn kein zerstörender Stress auf sie einwirkt.

Für Laborversuche, insbesondere für die Lokalisierung und Klonierung von Genen ( 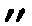 gene tagging/transposon tagging") und für deren wirtschaftliche Anwendungen, ist ein im Evolutionstempo ablaufender Prozeß (ca. 1 - 2 % natürliche Variation der DNA in der Gesamt-Erbinformation der Biosphäre pro 1 Mio. Jahre, nur ein Teil davon geht auf das Konto der Transposition) zu langsam und zu aufwendig.

Es mangelte bisher nicht an Versuchen, die Transposonen durch physikalische, chemische und biologische Einwirkung in Bewegung zu bringen: Radioaktive, Röntgen- und UV-Bestrahlung, chemische Zellgifte und virale Infektionen erhöhen die Transpositionsrate und können zur Sichtbarmachung des Phänomens dienen. So waren die radioaktiven Niederschläge aus amerikanischen Kernwaffenexplosionen die Auslöser für das Erscheinen instabiler Pigmentierungsmuster am Maiskorn (En/I bzw. Spm System) und von ebenfalls transposonenbedingten Chlorophylldefekten in Blättern.

Jedoch ist die Verwendung dieser Mittel zur Transposonenaktivierung von schweren degenerativen Veränderungen der Organismen begleitet, was ein Monitoring und die gezielte systematische Verwendung wesentlich erschwert.

Ein Mittel zur zerstörungsfreien Erhöhung der Transpositionsrate ist aus der DE 19543898 C1 bekannt.

Aufgabe der Erfindung ist es, die natürlicherweise extrem seltene genetische Transposition in einen effizienten, steuerbaren, technisch verwertbaren Prozeß überzuführen und dabei Zelle, Organ und Organismus voll funktionsfähig zu halten.

Gelöst wird diese Aufgabe durch die Verwendung von Aluminium gemäß Patentanspruch 1. Die Unteransprüche beschreiben vorteilhafte Ausgestaltungen der Erfindung.

Dies wird durch die Behandlung der Organismen mit anorganischen und organischen Aluminiumverbindungen erreicht.

Völlig überraschend war nun die Feststellung, daß Aluminiumverbindungen diese technische Aufgabe ebenfalls und in zahlreichen Fällen vorteilhafter erfüllen können. Dies ist umso erstaunlicher, als es sich bei Bor um ein Nichtmetall handelt, während hier derartige genetische Effekte dem Metall Aluminium zugerechnet werden können. Unter physiologischen Bedingungen bestehen zwischen beiden Elementen fundamentale Unterschiede; ersteres wirkt anionisch, letzteres kationisch.

Aluminiumverbindungen bringen gegenüber Bor den Vorteil ihrer geringeren Toxizität und der größeren Toleranzbreite, besonders im Veterinär- und Humanbereich. Im Gegensatz zu Bor können Aluminiumverbindungen im Veterinär- und Humanbereich in Grammengen administriert werden.

Weiterhin ist ein erfolgreicher Einsatz von Transposonen z.B. als genetische Werkzeuge ( 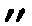 transposon tagging") ebenfalls nur durch eine signifikante Erhöhung der Transpositionsrate wirtschaftlich.

Im Gegensatz zur bisherigen biochemischen Negativrolle erfüllt das Aluminium in der erfindungsgemäßen Anwendung einen gewünschten Effekt.

Besondere Vorteile der Erfindung sind:
- drastisch verbesserte Effizienz bei der gezielten Suche nach einzelnen Genen in nichtsequenzierten Genomen mit Hilfe von Transposonen ( 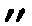 gene tagging")
- verstärkte transposonen-induzierte Mutation mit anschließen-der Selektion gewünschter Mutanten: beschleunigte Züchtungserfolge
- beschleunigte Entfernung funktionshemmender Transposonen aus den Genomen von Sorten und Arten zwecks Aktivierung von Genen für wirtschaftlich interessante Merkmale, z.B. zur Erhöhung ihrer Resistenz gegen chemische und biologische Noxen und gegen Infektionen
- gezielter Gentransfer durch verstärkt mobilisierte transponierbare Elemente; beispielsweise selektive Inhibierung viraler Zyklen, tumoraler Zellproliferationen und ggf. apoptotischer Selbstzerstörungsmechanismen.

Es wurde festgestellt, daß überraschenderweise durch Applikation von bioassimilierbaren (= bioverfügbaren) Aluminiumverbindungen in geeigneten Dosierungen die Excisionsrate von transponierbaren Elementen um Zehnerpotenzen erhöht werden kann, ohne daß es zu zellzerstörenden Nebeneffekten kommt.

Dies ist umso bemerkenswerter, als im bisherigen Stand der Technik den Aluminiumverbindungen keine sinnvolle physiologische Funktion zugeordnet werden konnte, und diese im Gegenteil sowohl im Pflanzen- als auch im Tierbereich ausschließlich mit toxischen und zerstörenden Effekten in Verbindung gebracht wurden. Als pflanzliches Beispiel sei die Wurzeldegeneration beim Waldsterben erwähnt, für die man die erhöhten Aluminiumsalzkonzentrationen in acidifizierten Böden hauptverantwortlich machte; ein Beispiel im Humanbereich für die physiologische Negativrolle von Aluminium sind die Aluminium-Depots in Alzheimer-Hirnen.

Es handelt sich hierbei um klassische, oft wiederholte Fehleinschätzungen im Stand des Wissens und der Technik, in der Weise, daß man den beobachtbaren Endeffekt eines komplexen Vorganges für den Verursacher hält. Der eigentliche Verursacher ist aber noch unbekannt, und die irrige Ansicht persistiert solange, bis der eigentliche Auslöser aufgefunden wird.

Im Gegensatz zur bisherigen biochemischen Negativrolle erfüllt das Aluminium in der erfindungsgemäßen Anwendung einen gewünschten Effekt.

Durch die erfindungsgemäße Applikation von Aluminium wird es möglich, die Häufigkeit der Transposition als Prozeß in die Nähe anderer fundamentaler genetischer Grundprozesse, wie
- der artenstabilisierenden allgemeinen Replikation und der Mutationen einschließlich der 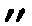 artenaufweichenden" viralen Aktivitäten,
- beides von Natur aus Prozesse von hoher Effizienz.

Das bis dahin dem statistischen Zufall überlassene Transposon wird zu einem kontrolliert einsetzbaren Werkzeug.

Weiterhin wurde festgestellt, daß Aluminiumverbindungen in anderer Weise auf die Transposonenmobilität einwirken als die ähnliche Effekte hervorrufenden Borverbindungen. Werden mehrere Transpositionsproteine kodiert, ist die Wirkung von Aluminium stärker als der entsprechende Bor-Effekt, z. B. bei der Transposonenfamilie TAM 1. Bei der Transposonenfamilie TAM 3, deren Aktivität auf einer einzigen Transposase beruht, ist Bor wirksamer als Aluminium. Eine Simultananwendung beider Transpositionsbeschleuniger Aluminium und Bor führt zu unerwartet hohen synergistischen Effekten führen und hat bei entsprechenden Dosierungen selektiv zellzerstörende Wirkung.

Wegen der Vielzahl der Anwendungen, die sich aus der erfindungsgemäß erzielten Erhöhung der Transpositionsrate ergeben, ist eine kurze Beschreibung des Standes der Technik notwendig.Struktur und Vorkommen von Transposonen.

Transposonen weisen eine universelle Standardstruktur auf, die von dem beherbergenden Wirtsgenom und seinen Sequenzen unabhängig ist. Von Wirtsseite ist keinerlei Homologie zwischen Excisionsort, Donor-, Transposonen- und Rezeptor-DNA (Integrationsort) erforderlich.

Transposonen bestehen aus einem Grundkörper, der als Mindestausrüstung die Gene zur Kodierung wichtiger Eigenfunktionsproteine enthält: in jedem Falle Transposase(n), ggf. Resolvase, Integrase, Repressionsproteine, reverse Transkriptase. Der kodierende einfache oder komplexe Zentralkörper wird beiderseits endständig von transposonenspezifischen umgekehrten Sequenzwiederholungen ( 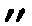 terminal inverted repeats" = TIR) flankiert. Mit Hilfe der Sequenzwiederholungen und der Transposase schneiden sich die Transposonen strangversetzt aus dem Ursprungslokus heraus und reintegrieren in Zielgene. Dabei werden in der Regel weniger weit entfernte potentielle Integrationsstellen bevorzugt. 50 % der Transpositionen erfolgen innerhalb des Chromosoms in geringer Entfernung vom Excisionsort, ohne das Centromer des betroffenen Chromosoms zu überschreiten, d.h. das Transposon verkürzt die extrachromosomale Phase auf ein Minimum und springt so schnell wie möglich wieder in das Genom hinein. Die übrigen 50 % fixieren sich in weiter entfernten Bezirken des Gesamtgenoms.

Gewisse Transposonen sind in der Lage, sich statistisch verteilt in beliebig weit entfernten Bezirken des Gesamtgenoms zu fixieren. Ihre extrachromosomale Lebensdauer ist hoch. Es handelt sich um Transposonen mit sehr langen terminalen Sequenzwiederholungen (TIRs), die ggf. noch von zahlreichen subterminalen Kopien gestützt sind, wie z. B. beim Mutator- Element im Mais (230 Bp im TIR). Solche transponierbare Elemente erlangen zusammen mit den zugehörigen Transpositionsproteinen eine so hohe Stabilität, daß sie unbehelligt von Nukleasen und Proteasen über den ganzen Genomapparat wandern.

Ganz allgemein sind Transposonen ubiquitäre genetische Einheiten, die in der gesamten lebenden Welt in allen Zellen vorhanden sind. Sie weisen zwischen den verschiedenen Arten einen hohen Grad an struktureller und sequentieller Homologie auf. Dadurch und dank ihrer funktionellen Autonomie sind zahlreiche Transposonen bei vollem Erhalt ihrer Transpositionsakitivität von einer Art auf andere Arten heterolog übertragbar. So springt das in Mais identifizierte Ac-Element nicht nur homolog in dieser Pflanze, sondern, nachdem es mit Hilfe der üblichen Genfähren dort eingetragen wurde, nachgewiesenermaßen auch heterolog in Tabak, Tomaten, Kartoffeln, Soja, Petunien, Arabidopsis. Konservierte Sequenzregionen pflanzlicher Transposonen finden sich u.a. auch in mobilen Elementen von Drosophila (P-Elemente, Hobo, Gypsy u.a.).

Ähnlich manchen Viren, die sowohl in Pflanzen als auch in Tieren aktiv werden, kann auch die transpositionale Information die Schnittstelle Pflanze/Tier überschreiten.

In den bisher untersuchten Genomen niederer und höherer Organismen sind Transposonen weit verbreitet. Dabei sind sie mehr oder weniger stabil integriert. Sie bilden Familien, die sich über zahlreiche Arten erstrecken können. Spezifisch ist nur das Transposon mit der Zahl seine endständigen, umgekehrten Sequenzwiederholungen, nicht aber die Wirts-DNA.

Die Universalität der Transposonen äußert sich auch in dem ubiquitären Vorhandensein ihrer Strukturelemente in den Genomen aller untersuchten Organismen, z.B. mindestens 3 % der DNA in den 4 Chromosomenpaaren von Drosophila; ein Auftreten mit hoher Frequenz in den bis jetzt bekannten mittel- und hoch-repetitiven Sequenzen des Humangenoms, die einen bedeutenden Prozentsatz seiner gesamten DNA ausmachen. Manche Genome können bis zu Millionen ein- und desselben Transposons enthalten. Der biologische Sinn derartiger Genomauffüllungen mit Hilfe von Transposonen ist noch nicht bekannt.

### Klassifizierung und Eigenschaften

Transposonen zeigen eine hohe Zahl von Variationen und Übergangsformen.
Dies äußert sich zunächst in ihrem schematischen Aufbau:
1.Die einfachste Gruppe bilden die Insertions-Elemente (IS) mit maximal 2000 Basenpaaren (Bp) z. B. im Genom und in den Plasmiden der Bakterien. Ihr Aufbau entspricht dem Grundprinzip der Struktur eines Transposons: Transposase-Gen(e) flankiert von kurzen inversen terminalen Repetitionen. Diese beiden Mindestbestandteile sind für den Transpositionsmechanismus essentiell und dürfen genetisch nicht verändert sein.
2.In komplexen Transposonen sind zusätzlich eingefangene oder eingebrachte Gene bzw. Gen-Fragmente enthalten. Sie sind für den Vorgang der Transposition nicht essentiell und in dem Element beliebig ersetzbar. Sie benutzen das Transposon als Vehikel für ihre Wanderung im Genom. Dabei werden Eigenschaften, z. B. Resistenzen gegen Antibiotika und Schwermetalle, übertragen (Tn bei Bakterien).
3.Bei zusammengesetzten Transposonen ist ein zentraler, kodierender Bereich von 2 Modulen der Gruppe 1 (IS-Elemente) flankiert. Auf diese Weise können ganze Gengruppen transponiert werden.

Es gibt 3 Transpositionsmechanismen:
1.Bei der einfachen exzisiven Transposition wird der Platzwechsel durch ersatzloses Ausschneiden des Elements aus der Ursprungsstelle initiiert. Die untersuchten Familien pflanzlicher Transposonen folgen im wesentlichen diesem Mechanismus.
2.Bei der replikativen Transposition werden vor dem Platzwechsel Kopien des Transposons angefertigt. Im Endzustand enthält dann sowohl die Ursprungs- als auch die Zielstelle das Transposon. Dieser Mechanismus ist bei Bakterien weit verbreitet.
3.Bei der Retrotransposition entsteht als Zwischenstufe ein RNA-Transkript des Transposons, welches mit Hilfe reverser Transkriptase - ähnlich den Retroviren - in die Transposonen-DNA rückübersetzt und anschließend in die Ziel-DNA integriert wird.

Die Mechanismen 2 und 3 führen zwangsweise kumulativ zu einer Erhöhung der Transposonenzahl im Gesamtgenom. Bei Mechanismus 1 erfolgt die Transposonenmultiplikation wahlweise je nachdem, ob das Transposon während der Replikation zwischen bereits replizierten bzw. noch nicht replizierten DNA-Strängen springt. Springt das Transposon während der Replikation von einem bereits replizierten Teil der Tochter-Strang-DNA in einen noch nicht replizierten Teil der Ursprungs-DNA, so wird es erneut repliziert und erscheint im anderen Tochter-Strang doppelt.

In jedem Falle erhöht sich auch hier im Laufe der Evolution die Zahl der mehr oder weniger definitiv fixierten transponierbaren Elemente.

Es wird angenommen, daß ausgedehnte, nicht kodierende Genomabschnitte in Eukaryonten durch den retrotransposonalen Mechanismus entstanden sind. Der Informationsgehalt aller Eukaryontengenome ist nämlich ungefähr gleich: 50 000 - 100 000 kodierende Gene. Die enormen Unterschiede in den Genomgrößen zahlreicher Arten resultieren aus der evolutionären Genomaufstockung meist mit Hilfe von Retrotransposonen. Sie ermöglichen das Längenwachstum und Strecken der Genome. Im Humangenom gehören die Lines-, Sines-, Alu-Familien dazu. Retrotransposonen werden stabil im Genom integriert und sind normalerweise nicht wieder excisierbar.

Bei Retrotransposonen deletiert ein zellulärer Reparationsmechanismus während der Integration die Basen Nr. 3 und 4 aus den TIRs. Damit ist das Transposon im Wirtsgenom immobilisiert, aber nicht inaktiviert. Über den retrotranspositionalen Mechanismus werden weitere Kopien erzeugt, die sich ebenfalls vermehren und die das Wirtsgenom progressiv auffüllen. Schließlich aggregieren die von zahlreichen Kopien in überkritischer Menge kodierten Transpositionsenzyme zu inaktiven Aggregaten. Die Transposition verliert an Präzision (defektive Elemente) und kommt schließlich zum Stillstand. In dem geschlossenen System der Zelle ist die Transposition selbstlimitierend. Solche Transposonen verbleiben als 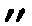 schweigende" Komponente in dem intakt gebliebenen Genom, werden mit diesem repliziert und an die Nachfolgegenerationen weitergegeben.

Mit der Retrotransposition identisch ist Teil 1 des retroviralen Zyklus. Retroviren sind nichts anderes als perfektionierte Retrotransposonen. Retroviren enthalten zusätzliche Gene zur Kodierung von Strukturproteinen für eine extrazelluläre Existenz. Retrotransposonen vermeiden es, durch zu starke Vermehrung ihre eigene Lebensgrundlage zu zerstören. Im Gegensatz dazu sind Retroviren vom dauerhaften Überleben ihrer Geburtszelle unabhängig. Die Wirtszelle muß lediglich bis zur Vollendung des viralen Zyklus aktiv bleiben. Retroviren sprengen den Zwang zur Selbstlimitierung, indem sie als infektiöse Partikel die von ihnen umfunktionierte, geschädigte und nicht mehr benötigte Zelle verlassen und sie der Zerstörung überlassen.

Wegen der engen Verwandtschaft von Retrotransposonen mit Retroviren, beinhaltet die Kontrolle der Retrotransposonen auch die

Einflußnahme auf retrovirale Infektionen und ist aus diesem zusätzlichen Grunde von weitreichender Bedeutung.

Die Transposition kann autonom erfolgen oder die Assistenz von Helferfaktoren erfordern. Es wird zwischen autonomen und defektiven transponierbaren Elementen unterschieden.
1.Autonome Elemente enthalten ein intaktes Transposase-Gen und unbeschädigte terminale Repetitionen (TIR). Sie sind voll funktionsfähig und können nachgewiesenermaßen heterolog in zahlreichen Organismen aktiv werden.
2.Defektive Elemente sind Transposonen, die ihre Autonomie verloren haben. Sie benötigen für ihre Genwanderung Helfertransposonen, z.B. ein autonomes Element der gleichen Familie, das für sie die Transposase kodiert. So ermöglicht das autonome Ac-Element im Mais den zahlreichen verschiedenen defektiven Ds-Elementen (einige 100) die Translokation. Derselbe Helfer-Effekt kann durch künstliche Expression einer geeigneten Transposase in der Wirtszelle erzielt werden.
3.Transposonen, die an den TIRs geschädigt sind, können nicht mehr springen, wohl aber in ihrem immobilisierten Zustand Transposase für defektive Elemente kodieren und diesen die verlorengegangene Mobilität wiedergeben.
4.Transposonen, die sowohl TIR- als auch transposasedefekt sind, verlieren jede Aktivität, d. h. sie können weder springen noch Transposase kodieren und verbleiben als 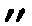 schweigende", scheinbar funktionslose Bestandteile im Genom.

An komplexen Transpositionsvorgängen ist ein Teil des Protein-Instrumentariums der eukariontischen Wirtszellen beteiligt: Mit Sicherheit nachgewiesen wurden die Rollen von Topo-Isomerase II und eines für die Bildung von Kruziformen verantwortliche Hu-Proteins, z. B. der Hefe.

Schließlich können Transposonen auch nach der 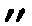 Virulenz" ihrer Ausbreitung unterschieden werden:
1.Im Normalfall ist das Transposon im Genom stabiler oder instabiler Mutanten vorhanden, die mit anderen Mutanten und dem Wildtyp koexistieren.
2.Invasive Transposonen können sich wie durch Ansteckung schlagartig verbreiten und eine gesamte Artenpopulation 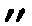 infizieren". So hat sich bei Drosophila das P-Element so virulent verbreitet, daß nur noch die Träger dieses Elements vorkommen. Der dieses Transposon nicht enthaltende ursprüngliche Wildtyp ist aus den natürlichen Populationen verdrängt worden. Das retrotransponierbare Element 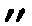 Gypsy" von Drosophila ist ebenfalls hochinfektiös.

### Transposonen und Viren

Wie bereits dargelegt, bestehen speziell zwischen Retrotransposonen und Retroviren ausgeprägte Analogien. Ganz allgemein können Transposonen mit 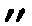 zellinternen Viren" verglichen werden. Mit den eigentlichen DNA-Viren gibt es begrenzte grundsätzliche Gemeinsamkeiten. Transposonen kodieren wie Viren zwar eigene Funktionsproteine (Transposasen), es werden aber keine Strukturproteine kodiert, da die Transposonen für ihre intrazellulare Translokation - im Gegensatz zu Viren - kein Kapsid zu bilden haben. Transposonen sind Viren in permanent eklyptischer Phase.
- Gemeinsam mit den DNA-Viren haben die Transposonen die beschriebene hohe Zahl an Variationen und Übergangsformen im Hinblick auf Aufbau, Mechanismus, Funktion und Ausbreitung.
- Als direkte Berührungspunkte zwischen beiden genetischen Klassen gibt es Viren, die sowohl als Virus als auch als Transposon funktionieren können.

Ein Beispiel dafür ist der virale Zyklus des Mu-Phagen mit wahlweise lytischer (mit Transposition) oder lysogener Zellinfektion (ohne Transposition). Die transpositionale Komponente löst hier die Aktivierung der im Wirtszellengenom ruhenden viralen Komponente aus.

Ein weiteres Beispiel ist das Retrotransposon 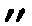 Gypsy" von Drosophila. Es stellt gleichzeitig das erste bisher bekannte Retrovirus der Insekten dar. Es ist hochinfektös.
- Im Allgemeinen gilt, daß Viren und Transposonen sich wechselseitig aktivieren können. Viren lösen transpositionale Aktivitäten aus, Transposonen aktivieren virale Zyklen.
- In ihrer primären Wirkung bringt die Insertion von Transposonen ähnlich der viralen Aktivität für die invadierte Wirtszelle und für den betroffenen Organismus zunächst nur Negativeffekte. Die Störfunktionen überwiegen. Die Aktivität der besetzten Wirtsgene bzw. ihrer Promotoren wird herabreguliert oder total inhibiert. Dadurch werden Genwirkketten beeinträchtigt oder unterbrochen. Die entsprechenden Genprodukte werden nicht oder nur in unzureichender Menge hergestellt.
- Transposonen, die in nicht kodierenden Regionen des Genoms eingeparkt werden, sind zunächst funktionslos und verursachen, ähnlich manchen zellintegrierten DNA-Viren, keine gravierenden Störungen, jedoch belastet ihre stille Anwesenheit zumindest die Energiebilanz der Wirtszelle.

### Transpositionale Hemmeffekte

Die Wirkung von Transposonen wird manifest, wenn
- das Transposon in exprimierenden Genen, in Promotoren oder in den Grenzkodierungsbereichen für Introns sitzt
- die Situation nach wenigstens einem generativen Zyklus von dem heterozygoten in den homozygot-blockierten Zustand übergeht.

Durch die Anwesenheit des Transposons im kodierenden Bereich wird das betroffene offene Leseraster ( 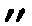 open reading frames" = ORF) gestört, die Anwesenheit in Introns kann den Spleißvorgang beeinträchtigen, die Anwesenheit im regulatorischen Bereich (z.B. Promotor) eines Gens kann dessen Expression beeinflussen. Als Folge werden Biosynthesen abgeschaltet, die Produktion normaler Transkripte wird herabreguliert oder blockiert. Dies äußert sich durch phänotypisch auffallende degenerative Effekte:
a) Bei Pflanzen bleiben beispielsweise Blütenfarbstoffe aus, Blattbezirke ohne Chlorophyll entstehen, Schrumpfungen an Früchten und Samen erscheinen, es kommt zu Stauchung und Zwergwuchs, Resistenzen brechen zusammen.
   - Bei Drosophila z.B. kommt es bei Besetzung der an der Expression beteiligten Gene zur Ausbildung flügelloser Mutanten.
   - Das Transposon wird auf diese Weise durch äußere Merkmale sichtbar und quantifizierbar. Die transpositionale Inaktivierung von Genen kann zum Tod von Zelle, Organ und Individuum durch Dysfunktion vitaler metabolischer Vorgänge führen.
   - Die Lokalisation eines Transposons in den die Zellproliferation kontrollierenden Genen kann Tumorbildung oder apoptotische Selbstauslöschung auslösen. Umgekehrt kann eine Transposonen-Insertion solche Prozesse auch zum Stillstand bringen.
   - Verläßt das Transposon das besetzte Gen, kann die entsprechende Revertante den Wildtypcharakter ganz oder teilweise wiedererlangen. Aus dem Wechselspiel zwischen Inser tion/Excision erwächst die für die Transposonen typische Instabilität der Mutation.
b) Ist das Transposon in den umfangreichen nicht kodierenden Bereichen eukariontischer Genome lokalisiert, bleiben die transpositionalen Hemmeffekte zunächst meist inapparent. Das Transposon sitzt symptomlos im Genom ähnlich den retroviralen Proviren und anderen persistent genomintegrierten DNA-Tumorviren.

### Leistungsabfall bei Hochzuchtsorten

Bei auf bestimmte Nutzeigenschaften hochgezüchteten Arten und Sorten kann es zu verstärkter Bildung und kumulativer Ansammlung von Transposonen kommen (Transposonenüberladung). Dies zeigt sich beispielsweise am Mais, der wichtigsten und am besten hochgezüchteten Nutzpflanze des Menschen: Hier wurden zuerst und in größerer Zahl die phänotypisch auffallenden Effekte ganzer Transposonenfamilien entdeckt (Ac/Ds; En/Spm; Mu). Ausführlich wurden bisher die transpositionalen Hemmeffekte auf diejenigen Genwirkketten untersucht, deren Produkte visuell besonders auffällig sind: Anthocyane, Flavone, Chlorophyll und Stärke.

Der Züchtungsvorgang führt zu einer unterschiedlichen Behandlung zwischen solchen Transposonen, die sich in exprimierenden Genen anreichern und so veränderte Merkmale ausprägen und solche, die sich ohne sichtbaren Effekt in nicht kodierenden Bereichen akkumulieren.

Selektion und Züchtung werden nach den vom Züchter gewünschten sichtbaren Merkmalen gesteuert und betreffen nur die Transposonen aus dem exprimierenden Bereich. Negative Merkmalsausprägungen werden zusammen mit den sie verursachenden Transposonen eliminiert, erhalten bleiben die 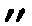 unsichtbar" integrierten Transposonen. In der Tat ist der nicht kodierende Genbereich vom züchterischen Selektionsdruck zunächst nicht betroffen, die Transposonen können dort ohne Kontrolle durch die Selektion akkumuliert werden. Mit züchterischer Entfernung vom Wildtyp kann die Transposonenansammlung in stillen Genombereichen wachsen. Im Epidemiefall kann es sich jedoch erweisen, daß man mit der Selektion auf gewünschte Leistungsmerkmale die Anfälligkeit gegen bestimmte Infektionen negativ beeinflußt hat, d.h. die Krankheit wurde mitgezüchtet:
- So entwickeln Wildpflanzen nach Viroidinfektionen kaum Symptome, während hochgezüchtete Monokulturen, Plantagen- und Gewächshauspflanzen empfindlich reagieren.
- Das CRP-Virus ( 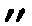 Cottontail Rabbit Pappiloma Virus") verursacht in Zuchtkaninchen maligne Tumore, während es für Wildkaninchen weitgehend inoffensiv bleibt.

Insgesamt betrachtet können Transposonen sowohl direkt sichtbare als auch verzögernd auftretende, schwerwiegende Negativeffekte verursachen.

Eine regulierbare, streßfreie Aktivierung von Transposonen in einem intakt bleibenden Genom ist in vielfacher Hinsicht von enormer wirtschaftlicher Bedeutung.

### Nutzeffekte

Wegen der negativen Primäreffekte stellt die Transposition eine potentielle Bedrohung für die Stabilität der Arten dar. In natürlichen Populationen wird daher die Transpositionsrate von der Zelle streng kontrolliert und extrem niedrig gehalten: Pro Zellgeneration finden nur 10⁻⁷ bis 10⁻⁴ solcher Ereignisse statt.

Umgekehrt beweist das universelle Vorkommen der Transposonen ihre grundsätzliche Notwendigkeit:

Die ständige und zwischen den Arten synchronisierte Neukombination von Genelementen durch Transposition ermöglicht und generiert die Diversität der DNA. Das Transposon ist der Motor der Evolution zu rasch formierten und stets besser angepaßten höheren Organisationsformen des Lebens.

Es wird angenommen, daß Transposonen während des Evolutionsprozesses die horizontale Übertragung genetischer Information sowohl zwischen verwandten als auch zwischen weit entfernten Arten, z.B. Pflanze und Tier vermitteln. Transposonen würden auf diese Weise die Evolution durch gegenseitige genetische Abstimmung der Lebewesen synchronisieren (konvergierende Evolution).

Als Gegenspieler zur genetischen Starre der Replikation sorgt die durch die Transposition generierte genetische Diversität bei abrupten Umweltveränderungen für die Erhaltung der Art durch das selektive Überleben der jeweils bestangepaßten Individuen aus einem breiten Spektrum von Mutanten.

Ein Beispiel für eine schnelle umweltbedingte Anpassung ist die transpositionsbedingte Resistenz von Mikroorganismen gegen Antibiotika und Schwermetalle. In diesen Fällen reichern sich Resistenzgene mit Hilfe der sie vehikulierenden Transposonen im Genom und in Plasmiden an.

Aufgrund der Universalität von Transposonen ist die Übertragung der Informationen nicht auf verwandte Spezies beschränkt und kann sich rasch zu einem allgemeinen Anpassungsphänomen entwickeln, das von einer großen Anzahl von Arten verwendet wird. Transpositionsbedingte genetische Veränderungen werden auf diese Weise zu einem Instrument der Eigenerhaltung für ganze Artenfamilien, die auf diese Weise abrupt veränderte Umweltbedingungen überleben, aber auch zu einem Mittel des Selektionskampfes zwischen den Subspezies (z.B. mittels 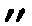 Killerproteinen" bei Mikroorganismen) einer Art.

Durch Insertion von Transposonen kann es auch zu Aktivierung von Genen der Wirtszelle kommen. Ein transposoneneigener Promotor liest über die Eigenfunktionsgene des integrierten Transposons hinaus in die Wirtszellen-DNA hinein und aktiviert somit angrenzende Gene aus dem Wirtsbereich, die normalerweise nicht oder kaum exprimiert werden.

### Transposonen als genetische Werkzeuge

In letzter Konsequenz ist das Transposon das streng kontrollierte genetische Werkzeug der Evolution, die weder explodieren noch erstarren darf.

Als ein solches Werkzeug ist es auch in der gentechnischen Forschung und Züchtung prinzipiell anwendbar.

Die Aufgabe besteht hier in der gezielten Suche nach einzelnen Genen in den immens langen Nukleotidsträngen der Chromosomen. Die selektive Gensuche ist von besonderer Bedeutung, weil sie eine wirtschaftlich akzeptierbare Alternative zu anderen Methoden der Genisolierung einschließlich der Totalsequenzierung von Gesamtgenomen darstellt. Letztere würde bei 1,5 Millionen eukariontischer Arten und einer mittleren Genomgröße von ca. 10⁹ Basenpaaren eine nicht realisierbare astronomische Arbeitsleistung erfordern (sequenzielle Identifizierung von 10¹⁵ Basenpaaren).

Mit Hilfe eines Transposons kann ein gesuchtes Gen (erkennbar an der instabilen Ausprägung des betreffenden Gens) etikettiert und, wenn die Nukleotidsequenz des Transposons bekannt ist, auch kloniert werden. Eine vorherige Kenntnis der primären Funktion des betreffenden Gens bzw. eine Kenntnis des entsprechenden Genprodukts ist dafür nicht erforderlich. Das einzige rekognitive Kriterium ist Anwesenheit des Transposons sowie seine Nukleotidsequenz. Das Verfahren wird als 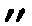 gene tagging" bzw. 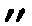 transposon tagging" bezeichnet und konnte bereits mit Erfolg eingesetzt werden.

Das 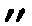 transposon tagging" ist damit die Methode der Wahl zur selektiven Klonierung beispielsweise von Resistenzgenen und Genen mit regulatorischen Funktionen sowie zur genomischen Defektsuche bei genetischen Krankheiten.

So wurden mit dieser Methode zunächst im Mais und im Löwenmaul eine Reihe von Strukturgenen und insbesondere von Genen mit regulatorischer Funktion erkannt und kloniert und zwar mit Hilfe der in diesen Pflanzen natürlicherweise vorhandenen und gut untersuchten Transposonen (z.B. Ac/Ds, En/Spm und Mu im Mais bzw. TAM 1 und TAM 3 im Löwenmaul).

Inzwischen ist es auch gelungen, einige der bekannten Transposonen des Mais und des Löwenmauls mit Hilfe geeigneter Genfähren in andere Pflanzenarten wie beispielsweise Tomaten, Kartoffeln, Petunien und Arabidopsis zu übertragen. Interessanterweise können sie auch im Genom dieser heterologen Systeme ihre Position wechseln und instabile Mutationen erzeugen. Dadurch ist das selektive Markieren und Klonieren von Genen auch in Pflanzenarten möglich geworden, die natürlicherweise keine bzw. keine dafür geeigneten Transposonen besitzen. Prinzipiell gesehen sind das 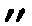 gene tagging" und damit die selektive Gensuche mittels Transposonen zu einer universell einsetzbaren Methode geworden. Erfolg oder Mißerfolg des 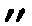 gene tagging" hängen allerdings in homologen wie in heterologen Systemen von der Transpositionsrate des verwendeten Transposons ab, die natürlicherweise extrem niedrig ist (ca. 1 : 10⁶).

In manchen Fällen wäre es vorteilhaft, das gewünschte Gen simultan durch 2 unterschiedliche Transposonen zu kennzeichnen. Dies stellt ein noch viel selteneres Ereignis dar. Das Warten auf ein statistisches Erfolgsereignis wird unerträglich lang.

Eine weitere Minderung der transpositionalen Erfolgswahrscheinlichkeit ergibt sich daraus, daß die an sich schon unwahrscheinliche Freigabe des Transposons mit der Integration in ein Ziel-Gen beendet werden soll, welches nur eines unter üblicherweise 100 000 eukariontischen Genen ist.

Das für die Etikettierung verwendete Transposon muß sich bei seiner Transposition gerade in dieses Gen integrieren. Die theoretisch konzipierte einmalige Methode der gewünschten Etikettierung eines bestimmten Gens war durch das bisher unüberwindliche Hindernis des Fehlens einer störungsfreien Aktivierung der Transposition kaum realisierbar (statistische Wahrscheinlichkeit ca. 1 von 10¹⁰ Individuen).

So mußten z.B. bei Pflanzen eine sehr hohe Zahl von Individuen angebaut und auf den Mutanten-Phänotyp hin untersucht werden.

Trotz großer Anstrengungen konnten auf diese Weise bisher nur einige wenige wirtschaftlich wichtige Gene kloniert werden, und dies unter ungeheurem Aufwand an Material, Zeit und Kosten.

Die Beseitigung dieses Hindernisses durch eine signifikante Erhöhung der Transpositionsrate ist deshalb für das 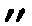 gene tagging" von enormer Bedeutung.

### Erfindungsgegenstand und Ausführungsbeispiele

Der Erfindungsgegenstand besteht in der überraschenden, dramatischen Erhöhung der Transposonenmobilität (Excision und Integration). Der Effekt wird streß- und zerstörungsfrei durch die Einwirkung bio-assimilerbarer Aluminiumverbindungen erzielt. Der Erfindungsgegenstand wurde am Beispiel der bekannten und weitverbreiteten Tranposonenfamilie TAM 1 untersucht und wird anhand der nachfolgenden Ausführungsbeispiele belegt. Durch die Wahl dieser Pilotfamilie wird das wichtigste bis jetzt experimentell untersuchte Transpositionssystem in die erfinderische Verbesserung einbezogen. Jedoch hat die Aluminiumaktivierung transposabler Elemente auch für andere bekannte Systeme universell Gültigkeit, so z.B. auch für die Transposonenfamilie TAM 3.

Das Transposon TAM 1 besteht aus 15164 Basen (B) und beeinflußt bei der hier verwendeten Mutante von Antirrhinum majus das niv-Gen, wo es beispielsweise 17 Basenpaare (Bp) entfernt von der TATA-Box des für die Chalkonsynthase kodierenden Gens integriert ist. Es wird von jeweils 2 perfekt invertierten Wiederholungssequenzen aus 13 Basen flankiert und erzeugt am Integrationsort eine 3 Basenpaare lange Sequenzverdopplung ( 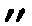 target site duplication" = TSD). TAM 1 kodiert zwei Transposasen. Aufgrund dieser kompletten Eigenausrüstung besitzt es eine perfekte Autonomie und kann - heterogen übertragen - auch in anderen Pflanzen springen.

Zur gleichen Familie gehören das transponierbare Element En/Spm im Mais, Tpm 1 in der Sojabohne und zahlreiche andere, die heterolog in andere Pflanzengenome implantiert und dort aktiv werden können. Bei der strangversetzten Excision hinterläßt das ausgeschnittene Transposon Sequenzduplikationen in Form von zusätzlichen Basenpaaren ( 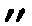 footprints") im zurückbleibenden und wieder ligierten Genabschnitt. Auf diese Weise ist der Excisionsort markiert. Das Gen der durch die Excision entstandenen Revertante ist nur selten (10 %) mit dem des Wildtyps identisch. Durch Punktmutationen kann das Genprodukt verändert werden. Sowohl die Transposition selbst, als auch die verbleibenden Sequenzveränderungen am Excisions- und am Integrationsort, stellen Glieder in einer Kette von neugebildeten stabilen oder instabilen Mutanten dar. Transpositionen erzeugen ein ganzes Spektrum von Mutanten.

Das Transposon TAM 3 - ebenfalls im Chalkonsynthase-Gen von Antirrhinum lokalisiert - besteht aus 3629 Bp, besitzt eine endständige perfekt invertierte Sequenzwiederholung (TIR) von 11 Basen und erzeugt eine 8 Basen lange 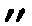 target site duplication" (TSD). Die Translokation wird autonom von einer einzigen selbstkodierten Transposase gesteuert. TAM 3 ist repräsentativ für eine andere weitreichende Familie mit gleichen oder ähnlichen Transpositionsparametern (Elemente Ac/Ds im Mais, Hobo in Drosophila).

TAM 1 ist bei den verwendeten instabilen Mutanten von Antirrhinum majus in dem die Chalkonsynthase (Schlüsselenzym) kodierenden Gen lokalisiert. Dadurch wird die Chalkonsynthese herabreguliert bzw. blockiert. Chalkone sind die Grundkörper für die Biosynthese der weitverbreiteten Stoffklasse der Flavonoide, u.a. auch der Anthocyane, die im Antirrhinum in Blüten und Blättern charakteristische Rotfärbungen hervorrufen.

Die Mobilität der Transposonen TAM 1 und TAM 3 ist in diesen Mutanten von Antirrhinum direkt mit dem Antocyangehalt und den Färbungen in Blüten und Blättern der Pflanzen korreliert. Diese stellen die beobachtbaren und meßbaren Parameter für die Transposonenaktivität dar:
- Den unbewegt im Chalkonsynthase-Gen fixierten Transposonen TAM 1 und TAM 3 entsprechen ungefärbte Blüten und niedrige Anthocyangehalte in allen Teilen der Pflanze.
- Der Excisionsvorgang erlaubt in den meisten Fällen die Reaktivierung der Genwirkkette zur Chalkonbildung, restituiert die Anthocyansynthese und manifestiert sich durch das Wiedererscheinen rotgefärbter Bezirke im Blüten- und Knospengewebe. Frühe Excisionsereignisse werden auf nachfolgende Zellengenerationen weitergegeben und erzeugen größere Farbbezirke; spätere Excisionen werden durch immer kleinere Tüpfelungen sichtbar. Auf diese Weise konnte die Transposition qualitativ (ja/nein), quantitativ (wie oft) und zellgenerationsdynamisch (wann) verfolgt werden.
- Wegen der extrem niedrigen Transposonenaktivität
   (normalerweise: 1: 10⁻⁶ pro Zellgeneration) sind die Blüten und Knospen der Antirrhinum-Mutanten von TAM 1 bzw. TAM 3 weiß mit gelegentlichen Farbtüpfelungen.

Erfindungsgemäß wird die natürlicherweise extrem niedrige Transposonenaktivität von TAM 1 durch Zufuhr geeigneter Mengen von bioassimilierbaren Aluminiumverbindungen um Zehnerpotenzen angehoben.

Dies zeigt sich in dramatischer Weise in dem verwendeten Experimentalsystem wie folgt:
- Der Anthocyangehalt des Blütengewebes steigt größenordnungsmäßig um den Faktor 2-3 an.
- Der gleiche Effekt wird in der unteren Epidermis der Blätter beobachtet. Diese sind grün bei Anwesenheit des Transposons (TAM 1 im Chalikonsynthase-Gen). Durch natürliche Excision treten selten rote Bereiche auf. Bei Anwendung von Aluminiumverbindungen werden Zahl und Größe der rotgefärbten Zonen drastisch verstärkt.

Die Applikation von Aluminiumverbindungen bewirkt also mittelbar oder unmittelbar eine Revertierung der transposonen-beladenen, instabilen Mutanten in ein Spektrum wildtyp-ähnlicher Formen.

Damit zeigt sich in unerwarteter Weise die genetische Wirksamkeit einer Verbindungsklasse, die in der Biochemie bisher in dieser Richtung nicht untersucht wurde.

In einer beispielhaften Ausführungsform zur Stimulierung der Transposonenaktivität durch bioassimilierbare Aluminiumverbindungen wurde wie folgt verfahren:

Samenkörner der instabilen Antirrhinum-Mutante (TAM 1) wurden jeweils unter üblichen Gewächshausbedingungen auf Blähton oder anderen inerten Substraten zur Keimung gebracht und auf 5 cm Länge angezogen.

Kontroll- und Versuchspflanzen wurden dabei mit Hoaghland-Nährlösungen behandelt. Danach wurden die Jungpflanzen in Hydrokulturtöpfen unter streng kontrollierten Bedingungen und bei regelmäßiger Erneuerung der Nährlösung weitergezogen. Die Aluminiumkonzentration (als Al(NO₃)₃ appliziert) betrug in den ersten 17 Tagen 5 ppm Al und danach 2,5 ppm.

Die Aluminiumapplikation kann zusätzlich oder allein auch folial erfolgen, vorzugsweise unter Zufügung eines Benetzungsmittels. Folial ist es möglich, beispielsweise komplex stabilisierte Aluminiumverbindungen auch partiell zu applizieren.

Die Auswertung erfolgte optisch halb-quantitativ durch Analyse der Färb- und Tüpfelungsmuster der Blütenknospen sowie nach Extraktion spektrophotometrisch durch Bestimmung der Anthocyanmengen in Blütenknospen und Blättern. Extrahiert wurde in bekannter Weise mit 1 % HCl in Methanol, gefolgt von einer HPLC Trennung (C-18 Säule, 5 % Ameisensäure in Acetonitril) und optischer Detektion bei 530 nm. Dabei ergab sich beispielsweise:
- Erfindungsgemäß aluminiumbehandelte Mutanten zeigten parallel zur applizierten Aluminiummenge und zur Dauer der Aluminiumeinwirkung progressiv flächendeckende Rotfärbungen in den Blättern und Blütenknospen.
- In üblicher Weise zeigten die Kontrollpflanzen der instabilen TAM 1 - Mutanten hingegen weitaus weniger Rotfärbungen in den Blättern und Blütenknospen.

Noch überzeugender war die physiko-chemische Anthocyan-Bestimmung:
- Der Farbstoffgehalt in den Blütenknospen der aluminiumbehandelten Pflanzen war bis zu 3 mal höher als in den Kontrollpflanzen.
- Besonders ausgeprägt war der Unterschied in den aus den Blättern isolierten Anthocyanmengen: Aluminiumbehandelte Pflanzen zeigten intensiv rot gefärbte Anthocyanablagerungen in der Epidermis der Blätter, was bei den Kontrollpflanzen nicht der Fall war. Physiko-chemisch konnte bei letzteren nur wenig Anthocyan nachgewiesen werden, während bei den Aluminiumpflanzen bedeutsame Mengen isoliert und identifiziert wurden: Im Vergleich zum Kontrollversuch stieg hier der Anthocyangehalt um das 15 - 20fache an.

Diese Befunde belegen die erfindungsgemäße bedeutsame Stimulierung der Transposonenaktivität bis hin zur vollständigen Entfernung aller Transposonen aus dem Ursprungslocus.

Gleichzeitig bewirkt die erfindungsgemäße Transpositionsbeschleunigung das rasche Auftreten eines breiten Spektrums neuer stabiler Mutanten, was einer Evolution im Zeitraffer-Tempo gleichkommt.

Ursache dafür sind die oben beschriebenen Mechanismen, insbesondere Footprint-Mutationen im Ursprungs-Gen und 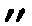 target-site-duplications" (TDS) im Ziel-Gen, die sich den durch die eigentliche Genwanderung bewirkten Effekten überlagerten. Auch das Transposon selbst kann bei der Wanderung mutieren (z.B. Deletionen, Richtungsinversion von Genen und/oder Promotern).

In allgemeiner Weise kann die Applikation der aluminiumhaltigen Lösungen nutritiv über Böden oder in Hydrokultur oder folial durch Versprühen oder über beide Wege erfolgen. Den Pflanzen werden im Boden oder sonstigen Nährmedien und/oder auf jede andere Weise bioassimilierbare, anorganische oder organische Aluminiumverbindungen im Rahmen der für die Spezies und Sorte geltenden Toleranzgrenzen zugeführt, wobei die Applikationsform jede Art von boden-, pflanzen- und allgemein bioverträglichen anorganischen und organischen Aluminiumverbindungen oder Mischungen daraus sein kann, vorzugsweise wäßrige Lösungen von Al³⁺-Ionen, wie von Aluminiumnitrat, Alaunsalzen und von organischen Aluminiumverbindungen und -komplexen, insbesondere von Aluminium-Chelatkomplexen, beispielsweise auch von Polyolen und Polyphenolen wie die verschiedenen Flavonoide, Anthocyane und Coumarine, die von sich aus als Pflanzeninhaltsstoffe weit verbreitet sind.

Die Applikationsform von Bor, bei gemeinsamer Applikation mit Aluminium, kann jede Art von boden-, planzen- und allgemein bioverträglichen anorganischen und organischen Borverbindungen oder Mischungen daraus sein, vorzugsweise wäßrige Lösungen von Borsäure, von Borarten, die ggf. neutralisiert werden, von organischen Borverbindungen, insbesondere von Boratchelatkomplexen mit Polyolen wie Glykol, Glycerin, Mannit oder Sorbit, mit Oxycarbonsäuren, wie Milch-, Wein- oder Zitronensäure oder in Form von Kohlenhydratkomplexen mit Glukose, Galaktose, Fruktose, Maltose, Laktose oder jeder anderen Art von Mono-, Di-, Tri- und Oligosacchsariden als glykosidische Liganden.

Die erfindungsgemäße Mobilisierung von Transposonen ist nicht auf den Pflanzenbereich beschränkt und kann im Rahmen der jeweiligen Verträglichkeit auch auf tierische Organismen, Mikroorganismen und Zellkulturen angewandt werden, wobei die hohe Verträglichkeit von tierischen Organismen für Aluminiumverbindungen von besonderem Interesse ist.

Überhaupt ist bei der Aluminiumapplikation vor allem im Tierbereich, bei Mikroorganismen und Zellkulturen den im jeweiligen physiologischen Milieu bereits natürlicherweise zirkulierenden Metaboliten, die als potentieller anionischer Partner bzw. Komplexbilder für das Aluminiumion infrage kommen, der Vorzug zu geben, wodurch ein invasiver Charakter der Aluminiumbehandlung vermieden wird. Dazu gehören auch geeignete Oxycarbonate, z. B. Citrat, Lactat, Acetotartrat u. a., die in zellulären Zyklen vorkommen. Gegebenenfalls können auch für einen speziellen Fall chemisch konstruierte Komplexpartner oder Käfigverbindungen verwendet werden.

Zur Stimulierung der Transposonenmobilität bei Zellkulturen in der Mikrobiologie sowie im Veterinär- und Humanbereich finden im wesentlichen die gleichen Applikationen unter strengerer Beachtung der jeweiligen Toleranzgrenzen Verwendung, ggf. wird die Wahl von der Anpassung an bestehende Puffersysteme mitbestimmt.

Wegen der Universalität der Transposition ist auch das erfindungsgemäße Monitoring der Transposonen in seinem Anwendungsbereich universell. Die dosierbare, zerstörungsfreie Freigabe der natürlicherweise genetisch streng versiegelten Transposonen löst eine ganze Serie von Anwendungen mit großem wirtschaftlichem Interesse aus. Sie erlaubt die Realisierung einer Vielzahl von Prozessen auf den Gebieten der Züchtung, Genetik, Therapie u.a., die bisher zwar theoretisch möglich, wegen der natürlicherweise extrem niedrigen Transpositionsrate jedoch nicht oder nur unter ungeheurem Aufwand durchführbar waren. Dies wird an weiteren Anwendungsbeispielen beschrieben.

Die neuen folgenden Beispiele erläutern die Verwendung von Aluminium näher.

### 1. Enttranspositionierung zwecks Sortenverbesserung

Ähnlich wie bei den untersuchten Mutantenlinien von Antirrhinum werden auch bei anderen Pflanzen und Sorten funktionshemmende Transposonen aus wirtschaftlich wichtigen Genen zerstörungsfrei entfernt, wenn die Pflanzen der erfindungsgemäßen Aluminiumbehandlung unterworfen werden. Zum Beispiel werden auf diese Weise bei der Sortenzuzüchtung Transposonen von einer hochstörenden Position in unschädliche Genomstellen weggeparkt. Die bis dahin durch die Anwesenheit des Transposons gehemmte Genfunktion (Resistenzen gegen Parasiten, gegen Umweltgifte oder eine sonstige positive Eigenschaft, Produktion gewünschter Pflanzeninhaltsstoffe) wird auf diese Weise gesteuert. Die erfindungsgemäße Aluminiumbehandlung von Pflanzen ist überall dort angetan, wo im Laufe eines Züchtungsvorganges Transposonen ungewollt und unbeachtet angesammelt wurden. Durch die Beseitigung der transpositionalen Hemmung werden Sorten von spezifischen Mängeln befreit, die während der Züchtung ungewollt miterworben wurden.

Durch die erfindungsgemäße Aluminiumapplikation können in Sorten vorhandene spezifische Mängel behoben werden. Zweckmäßigerweise ist eine solche Aluminiumbehandlung in räumlich begrenzten Versuchsböden durchzuführen.

### 2. Früherkennung von Sorteninstabilitäten

Durch die erfindungsgemäße erhöhte Aluminiumzugabe werden transposonenbedingte Instabilitäten in Sorten im Zeitraffertempo aufgespürt.

Der normale Prüfungsgang z.B. einer Pflanzensorte ist ein aufwendiger und zeitraubender Vorgang, der sich über zahlreiche Jahre, Boden- und Klimazonen erstreckt. Aluminium erlaubt die frühzeitige Erkennung eventuell vorhandener Transposonen, die sich negativ auf die Stabilität einer Sorte auswirken können. Transposonenbedingte Mängel und Anbauerscheinungen, die natürlicherweise irgendwann im Laufe einer Streßsituation auftreten und die Qualität einer Sorte in Frage stellen, werden frühzeitig und beschleunigt aufgespürt. Dem normalen Prüfungsgang vorgeschaltet werden durch diese Aluminiumdiagnose Fehlinvestitionen in der Arten- und Sortenzüchtung vermieden.

Aluminium erlaubt auf diese Weise die Früherkennung von Sorteninstabilitäten.

### 3. Auffinden neuer Transposonen

Bis jetzt sind nur wenige Transposonen bekannt. Zum Beispiel sind in vielen Pflanzen noch keine natürlicherweise darin vorhandenen aktiven Transposonen gefunden worden. Will man Transposonen als genetische Werkzeuge verwenden, müssen Fremdtransposonen heterolog mittels Genfähren in das Genom eingeführt werden. Durch die erfindungsgemäße Mobilisierung eventuell natürlich in der entsprechenden Art vorhandener Transposonen, werden diese erkannt und für die beschriebenen Anwendungsbereiche verfügbar. Die homologe Verwendung von Eigentransposonen ist in vielen Fällen einfacher, sicherer und kostengünstiger als ihre heterologe Insertion in das gewünschte Artengenom.

Aluminium erlaubt die Erkennung und Verwendung von Faktoren, die instabile Mutationen verursachen.

### 4. Beschleunigung der Mutation als gewünschter Effekt

Durch die erfindungsgemäß beschleunigte Transposonenbewegung wird die Mutantenzahl drastisch erhöht.

Jede Excision und Integration eines Transposons ist mit Sequenzduplikationen im Wirtsgenom verbunden ( 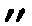 footprints" bzw. 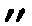 target site duplications"), die ganz, teilweise oder nicht erhalten bleiben.

Auch das aktivierte Transposon selbst kann mutieren. Dies bewirkt eine Kaskade genetischer Veränderungen.

Die erhöhte Aluminiumzugabe führt zu schnelleren und breiter gefächerten Mutationen, aus der die Individuen, die Träger der gewünschten Verbesserung sind, rasche und besser selektioniert werden können.

Die Erfindung stellt ein Verfahren zur beschleunigten Erlangung neuer pflanzlicher, tierischer und mikrobiologischer Sorten dar.

**5.** 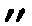 **gene tagging" als ökonomischer Prozeß**

Die Verfügbarkeit wirtschaftlich wichtiger Gene ist ein allgemeines Bedürfnis auf den Gebieten Züchtung, Genetik und Therapie. Zahlreiche prinzipiell mögliche Verfahren (z.B. somatische Gentherapie) waren nicht ausführbar, weil die implizierten Gene nicht charakterisiert sind.

Durch die aluminiuminduzierte Erhöhung der Transposonenmobilität wird das selektive Auffinden von Genen mit Hilfe von 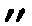 gene tagging" um Zehnerpotenzen rentabler, rascher und billiger.

Beispielsweise wird dadurch die systematische Suche nach Genen für Resistenzen gegen biotischen und abiotischen Streß, für die pharmazeutisch gewünschte, gezielte Produktion aller Art von Metaboliten und für die Verbesserung der inneren und äußeren Qualität landwirtschaftlicher und aller anderen Arten biologischer Produkte möglich.

**6.** 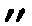 **gene tagging" in Gewebekulturen**

Die erfindungsgemäße hohe Effizienz der Transposition rückt auch ihre Anwendung in Kulturen tierischer und menschlicher Zellen in den Bereich der Möglichkeiten. Im Humanbereich können durch effizientes 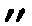 gene tagging" Ursachen von und Therapien für Krankheiten gefunden werden, die genetisch (mit)bedingt sind. Etwa 1500 humane Erbkrankheiten sind bisher genetisch identifiziert. Der Großteil ihrer ständig steigenden Zahl ist unaufgeklärt. Jeder Mensch trägt ca. ½ Dutzend schwerwiegender genetischer Defekte mit sich. Aluminiuminduziertes 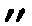 gene tagging" erlaubt die Entwicklung rascher Routinetestmethoden.

### 7. Gentransfer mit Hilfe aluminiumaktivierter Transposonen

Bei genügend hoher Transpositionsfrequenz können Transposonen in effizienter Weise als Genfähren verwendet werden. Bei den bisherigen Methoden erfolgt der Gentransfer weitgehend unspezifisch unter Bildung einer Vielzahl nicht vorhersehbarer stabiler und instabiler Mutanten. Bei der Verwendung von aluminiumaktivierten Transposonen, die mit einem zu transportierenden Gen beladen sind, läßt man das Transposon so lange springen, bis es an der gewünschten Stelle angekommen ist. Auf diese Weise kann ein in einer anderen Sorte gefundenes Resistenzgen in das Genom einer Hochleistungssorte implantiert werden, der die von diesem Gen kodierte Eigenschaft fehlt.

### 8. Gezielte Aktivierung von Genen (Transposon als Transaktivator)

Ein Transposon mit einem transposoneneigenen oder einem eigens dazu eingepflanzten, starken Promoter, z.B. 34S, wird in der Nähe und in der richtigen Leserichtung eines normalerweise schwach oder nicht exprimierten oder durch pathogene Vorgänge inaktivierten Wirtsgens insertiert. Der transposoneneigene Promoter liest über die Eigenfunktionsgene hinaus und (re)aktiviert das anvisierte Wirtszellengen. Voraussetzung dafür ist eine erhöhte Transpositionsrate, die wieder erfindungsgemäß durch Aluminium erzielt wird.

### 9. Gezielte Inaktivierung von Genen

Unerwünschte Genexpressionen werden erfindungsgemäß durch gezielte Implantation von Transposonen unterdrückt. Voraussetzung dafür ist, daß die Transposonen in genügender Anzahl zur Verfügung stehen, was durch eine hohe Mobilität erreicht wird.

Von besonderer Bedeutung ist dabei die selektive Unterbrechung viraler Zyklen und der Proliferation tumoraler Zellen sowie die Expression von Proteinen, die apoptotische Selbstauslöschung der Zellen auslösen ( 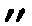 Transposonentherapie")

In einer speziellen Ausführungsform wird die Konstruktion und Verwendung von defekten transposablen Elementen (DTE) vorgeschlagen. Diese sind zur autonomen Transposition unfähig und üben in der normalen Zelle (mit oder ohne Aluminium) keine Hemmfunktionen aus. Ihre Aktivität ist von zellulären Hilfsfunktionen in der Weise abhängig, daß sie beispielsweise nur in virusinfizierten Zellen aktiv werden können. Aluminium leistet hier die erfindungsgemäße Excisionshilfe, die zur Erlangung einer genügend hohen Zahl hemmender Integrationsereignisse notwendig ist.

Zur selektiven Inhibierung retroviraler Zyklen, z.B. bei Leukämie- und HI-Viren, wird in einer besonderen Ausführungsform die Verwendung erfindungsgemäß aluminiumaktivierter defektiver retrotransponierbarer Elemente (DRTE) vorgeschlagen.

In derartigen, helferabhängigen Elementen ist das offene Leseraster (ORF) zur Expression der für die Retrotransposition notwendigen reversen Transkriptase (RT) defekt.
a) Um überhaupt zu springen, benötigt das Element eine transposonenexterne Helferfunktion. Diese wird vom Gen Pol des zu bekämpfenden Retrovirus geliefert, das in den infizierten Zellen eine große Menge reverser Transkriptase (RT) erzeugt.
b) Um oft zu springen und therapeutisch effizient zu sein, benötigt das Element die erfindungsgemäße Aktivierung durch Aluminiumverbindungen. In infizierten Zellen springt das gentherapeutische Transposon solange, bis
   - der virale Zyklus durch Transposoneninsertion inhibiert ist
   - die infizierte Zelle durch übermäßige Chromosomenauflockerung abstirbt und keine Viren mehr erzeugen kann. Das Retrotransposon hat durch hyperfrequente
   - Transpositionsvorgänge und exzessive Kopienzahl seine eigene chromosomale
   - Lebensgrundlage zerstört. Damit wird zellselektiv auch der virale
   - Ko-Infektor neutralisiert.

Gesunde Zellen werden davon nicht betroffen, da das defektive Transposon nicht über die notwendige Menge an Retrotransposase (RTP) verfügt und virusabhängig ist.

Während z. B. die bisherigen Anti-HIV-Strategien hauptsächlich auf die Total-Suppression der reversen Transkriptase (RT) hinausliefen, was schlechthin unmoglich erscheint, da wahrscheinlich die meisten Zellgenome zumindest einige Moleküle RT als evolutionäres Relikt mitkodieren, wird im Gegensatz dazu die RT in diesem System zur Abwehr des sie erzeugenden Virus benutzt: je mehr Virus, desto mehr RT, desto mehr erfindungsgemäß aluminiumstimulierte Transpositionsereignisse, desto effizienter die Virusabwehr durch immer häufigere Transposonensprünge.

Das Virus leitet auf diese Weise seine selektive Selbstzerstörung ein (Suizidstrategie).

## Patentansprüche

1. Verwendung von Aluminium in Form von bioassimilierbaren Aluminiumverbindungen zur Regulierung der genetischen Transposition bei Pflanzen, Tieren, deren Zellkulturen und bei Mikroorganismen.

2. Verwendung von Aluminium nach Anspruch 1, wobei mit Hilfe der Regulierung der genetischen Transposition bei Pflanzen und deren Sorten vorhandene spezifische Mängel behoben werden.

3. Verwendung von Aluminium nach Anspruch 1 zur Früherkennung von Sorteninstabilitäten.

4. Verwendung von Aluminium nach Anspruch 1, wobei die Regulierung der genetischen Transposition zur Erkennung von Faktoren, welche instabile Mutationen verursachen, ausgenutzt wird.

5. Verwendung von Aluminium nach Anspruch 1 zur Beschleunigung der Züchtung neuer Sorten.

6. Verwendung von Aluminium nach Anspruch 1 zum 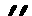 gene tagging".

7. Verwendung von Aluminium nach Anspruch 1 zum Gentransfer.

8. Verwendung von Aluminium nach Anspruch 1 zur Aktivierung von Genen.

9. Verwendung von Aluminium nach Anspruch 1 zur Inaktivierung von Genen.

10. Verwendung von Aluminium nach einem der Ansprüche 1 - 9 zusammen mit Borverbindungen.
